(19) Europäisches Patentamt

European Patent Office

Office européen des brevets

(11) **EP 3 477 653 A1**

(12) **EUROPEAN PATENT APPLICATION**

(43) Date of publication:
01.05.2019 Bulletin 2019/18

(51) Int Cl.:
*G16H 40/63* (2018.01)    *G16H 20/30* (2018.01)

(21) Application number: 17198691.2

(22) Date of filing: 26.10.2017

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventors:
• **RISPENS, Sietse Menno**
**5656 AE Eindhoven (NL)**
• **ANNEGARN, Janneke**
**5656 AE Eindhoven (NL)**

(74) Representative: **de Haan, Poul Erik**
**Philips International B.V.**
**Philips Intellectual Property & Standards**
**High Tech Campus 5**
**5656 AE Eindhoven (NL)**

(54) **MONITORING ACTIVITY OF A PERSON**

(57)    Presented are concepts for monitoring a person. One such concept employs event data relating to a plurality of occurrences of an event, the event data comprising, for each event occurrence, information representative of a detected value of a property of at least one of: the person; and an object controllable by the person. The received event data is processed to determine a first summary value for a first set of event occurrences that occurred during a first time period and to determine a second summary value for a second set of event occurrences that occurred during a second, different time period. Also, first and second measures of confidence in the first and second summary values are determined, respectively. A change in a capability or activity of the person is determined based on the first and second summary values and the first and second measures of confidence.

FIG. 3A

**Description**

FIELD OF THE INVENTION

This invention relates to monitoring activity of a person and more particularly to monitoring for a change in a capability or activity of a person.

BACKGROUND OF THE INVENTION

Functional assessment or monitoring of a person's health status, physical abilities, mental abilities, or recuperation after injury, hospitalization and treatment is of primary concern in most branches of medicine, including geriatrics, rehabilitation and physical therapy, neurology and orthopaedics, nursing and elder care.

Investigations have found that an individual's functional ability is actually environment-specific, since function increases when subjects are in familiar surroundings due to reduced confusion. Also, one-time assessment of function does not allow for assessment of variability of functional performance over the course of a day or several days, nor does it allow for assessment of change which is important in determining the adequacy of certain clinical services and treatments (such as rehabilitation) following functional loss.

A consensus therefore exists that it is preferable to assess or monitor independent functioning of a person at their home or within familiar surroundings.

A level of independent function is commonly indicated by the quality in which Activities of Daily Living (ADLs) are performed. ADLs refer to the most common activities that people perform during a day. Therefore, a reduced quality in the ADLs can be an indicator for care needed. For example, an anomaly in the regular performance of one or more ADLs can serve as warning for special attention.

Devices and systems have been developed to monitor the ADLs of individuals as they live independently in their own home or within familiar surroundings. For example, one such known system for detecting activities of daily living of a person system comprises three main components: (i) a sensor system that collects information about the person's activities and behaviours; (ii) an intelligence (or information processing) system that interprets the sensor signals for determination of ADL behaviour; and (iii) a user interface system that enables care givers to inspect the interpreted (processed) information. The intelligence system typically makes use of computational techniques known in the art as artificial intelligence. The system may be supported by conventional technologies for data collection, transmission, and storage.

In practice, however, a major difficulty is encountered by the wide range of variations that can happen in actual care cases. Since there are so many possible circumstances, situations, environment layouts and contexts that can occur in daily life, it is common to employ numerous sensors in an attempt to capture enough information about a person's activities and/or environment to enable identification of specific activities. This, however, typically increases complexity and/or costs and may therefore be undesirable.

The ever-increasing complexity in striving to cover all possible contexts and situations requires more elaborate and detailed information to be collected, processed, interpreted and/or communicated. Accuracy, or the amount of relevant information generated, may also be reduced by trying to cater for a large number of alternate situations. For example, although many situations may not be relevant to a monitored person, these irrelevant situations may still be accounted for and selected by the system, thus providing erroneous responses.

Also, a monitored person's physical and/or mental abilities may vary (e.g. decline or reduce) over time. As a result, it can be difficult to determine when some form of assistance, help of supervision may be needed. Natural variability and noise in captured information can therefore make it difficult to distinguish between significant changes (e.g. that can represent an unexpected or unusual variation in a monitored person's physical and/or mental abilities) and smaller changes that may be the result of natural variation or detection noise.

SUMMARY OF THE INVENTION

The invention aims to at least partly fulfil the aforementioned needs. To this end, the invention provides devices, systems and methods as defined in the independent claims. The dependent claims provide advantageous embodiments.

There is provided a system for monitoring activity of a person, wherein the system comprises: a signal interface adapted to receive event data relating to a plurality of occurrences of an event, the event data comprising, for each event occurrence, information representative of a detected value of a property of at least one of: the person; and an object controllable by the person; a data processing unit adapted to process the received event data to determine a first summary value for a first set of event occurrences that occurred during a first time period and to determine a second summary value for a second set of event occurrences that occurred during a second, different time period; a data confidence unit adapted to determine first and second measures of confidence in the first and second summary values, respectively; and a monitor unit adapted to determine a change in a capability or activity of the person based on the first and second summary values and the first and second measures of confidence.

Proposed is the concept for monitoring a physical or mental capability of a person by identifying a change in the physical or mental capability using detected values of a property of the person or an object manipulated by the person. By using summary values for sets of event occurrences from different time periods, along with measures of confidence in the summary values, a meaningful change in the parameter may be identified. For example, by determining a measure of confidence (e.g. reliability measure) in the detected values and/or the summary values, embodiments may enable the identification of changes in a parameter of an event that are meaningful and representative of underlying changes in a person's physical or mental capability, as distinct from changes expressing natural variability over time or estimation noise. This may help to reduce a number of false-positives (i.e. inaccurate or incorrect instances) and provide more accurate monitoring. Further, embodiments may be used for evaluation purposes, for example to assess if a subject shows a significant improvement in a physical or mental capability. Detected changes (e.g. cumulative weight losses, reaction times, movement speeds, applied forces, etc.) for a predetermined timeframes (e.g. a week or month) may also be used to estimate a trend in a physical or mental capability of the person for a current, following and/or preceding timeframe.

For example, weight measurements can be indicative of food or liquid consumption, and differences between consumption and/or weight measurement(s) may be used to identify pattern in a person's food/liquid consumption and/or weight variations that may, in turn, be used to infer a trend in a physical or mental capability of the person. For instance, sudden weight loss/gain outside of acceptable range of an established trend may indicate the occurrence of a problem/issue with a physical capability (such as walking or running), or, worse, the health, of the person.

A change in a physical or mental capability of a person may therefore be inferred using a single sensor (e.g. a weight scale) at a single location (e.g. a toilet). This may help to reduce associated cost and/or complexity of a monitoring system. For example, conventional ADL monitoring systems may be adapted to detect or monitor eating and drinking by employing sensors installed on a refrigerator (e.g. an open-close sensor), sensors installed in cupboards/drawers holding cutlery/food, power sensors on cooking equipment, presence sensors for detecting a user's presence in kitchen; a pressure sensor installed in a seat of a dining table, etc. Conversely, embodiments may avoid the need for multiple sensors (and complex signal processing of their respective signals) and may instead simply employ a single value (e.g. weight) sensing arrangement.

Embodiments may be based on a proposal to determine the reliability or minimal detectable change of a summarized feature individually, based on a collected set of events for one subject (e.g. a person or individual), while typically the reliability is based on comparison of multiple measures of features values for a group of participants.

By way of example, when monitoring a mobility of a person in their daily life, summarized features of activity events can be used, such as speed and regularity of walking, or peak-power or duration of chair rises. The summarizing of features may be achieved using one or more mathematical functions like the mean, median or 10th or 90th percentile of a feature's estimates over a number of events. A 95% confidence interval of the summarized feature for a specific individual may then be determined based on the distribution of a feature over that individual's events. This may account for estimation noise and natural variation over the summarised period, for example a day. The confidence interval may, for example, be determined as the standard error of the summarising function, for example the standard error of the mean (when the summarizing has employed the mean value). Further, to account for longer term variations, the mean value of the summarised values of several consecutive days may be determined. The 95% confidence interval of this mean value may then be determined in two ways: (1) by combining the confidence intervals of the individual days; and/or (2) by the standard error of the mean of the daily summary values. The higher of the two values may be taken as a best guess of the confidence interval of the feature value.

Accordingly, in some proposed embodiments, the data processing unit may be further adapted to determine a first characterising value for the first and second sets of event occurrences based on the first and second summary values. The data processing unit may then be further adapted to process the received event data to determine a third summary value for a third set of event occurrences that occurred during a third time period, to determine a fourth summary value for a fourth set of event occurrences that occurred during a fourth time period, and to determine a second characterising value for the third and fourth sets of event occurrences based on the third and fourth summary values. Also, the data confidence unit may be adapted to determine third and fourth measures of confidence in the third and fourth summary values, respectively. The monitor unit may then be adapted to determine a change in a capability or activity of the person further based on the first and second characterising values and the first to fourth measures of confidence. A characterising value may, for example, be calculated based on an average of summary value and/or a measure of deviation of summary values (e.g. a percentile range of the summary values). A characterising value may therefore be calculated in a similar manner to how a summary value is calculated. In this way, the same processes or hardware used to determine summary values may be used to determine a characterising value. Accordingly, a characterising value may be understood as representing an expected or typical value from a plurality of summary values (and their associated time periods).

By obtaining a characterising value for summarised values of a plurality of time periods, longer term (e.g. slower) variations may be assessed. This may be thought of as employing a 'second level' of summarising, since it may employ a concept of summarising (e.g. averaging) a plurality of summary values (so as to provide a characterising value of summary values for multiple time periods). For instance, where a summary value is determined for each day of a week, a characterising value (e.g. average) for the week may be determined from the summary values of each day of the week. Using such an approach, characterising values for different weeks may be compared, and this may be done taking account of the measures of confidence in the summary values that have been used.

Accordingly, proposed embodiments may be employed to identify deviating daily values by comparing event occurrences between days, and/or may identify slower changes (e.g. over weeks or months) by comparing characterised daily summary values over longer time periods (e.g. daily summary values averaged over different weeks or months). By employing such first and second levels for summarising of data, variations due to noise and/or expected trends can be filtered out so as to enable identification of meaningful changes over time periods of interest.

Further, the monitor unit may be adapted to compare the first and second characterising values to obtain a primary comparison result; to generate a first weighted characterising value based on the first and second summary values and the first and second measures of confidence; to generate a second weight characterising value based on the third and fourth summary values and the third and fourth measures of confidence; to compare the first and second weighted characterising values to obtain a secondary comparison result; and to determine a change in a capability or activity of the person based on the primary and secondary comparison results. Simple mathematical functions may therefore be employed, enabling straight-forward and reduced-complexity implementation.

To assess if a new value for the summary feature indicates a significant (e.g. notable, unexpected or substantial) change (e.g. improvement or deterioration), a measure of overlap between confidence intervals of the new and previous values may be used. For example, if they do not overlap, it may indicate that the change represents a change in a person's mobility. If the confidence intervals do overlap, the feature change may be the effect of natural variation, expected variation or estimation noise.

Embodiments may therefore be able to distinguish between substantial changes that represent an actual change in a person's physical or mental capability, and smaller changes that may be the result of natural variation or feature estimation noise.

Improved (e.g. more accurate) monitoring of a person's physical or mental capability may therefore be facilitated by analysing changes in one or more parameter values of an event (e.g. usage of an item of furniture, or duration of standing up). Embodiments may also be employed to infer a trend from detected changes in events.

Proposed embodiments may therefore be of particular relevance to ADL monitoring since, for example, it may assist in interpretation of a variation of capability or activity of a person over time. Proposed concepts may also facilitate signalling improvement of mobility features in coaching applications. This may be beneficial for identifying a significant decline in the mobility of a person, for example.

The data processing unit may be adapted to determine the first summary value based on an average value of the detected values of the property for the first set of event occurrences, and to determine the second summary value based on an average value of the detected values of the property for the second set of event occurrences. For example, the average value may comprise the mean, mode or median value of the detected values of the property. In some embodiments, the data processing unit may be adapted to determine the first summary value based on a percentile range of the detected values of the property for the first set of event occurrences, and to determine the second summary value based on a percentile range of the detected values of the property for the second set of event occurrences. Simple mathematical functions may therefore be employed, enabling straight-forward and reduced-complexity implementation.

The data confidence unit may be adapted to determine the first measure of confidence based on a standard deviation of the detected values of the property for the first set of event occurrences and to determine the second measure of confidence based on a standard deviation of the detected values of the property for the second set of event occurrences. For example, the data confidence unit may be adapted to determine the first measure of confidence based on a standard error of the first summary value and to determine the second measure of confidence based on a standard error of the second summary value. Known and relatively simple mathematical functions may therefore be employed to determine a measure of confidence. This may reduce the complexity and/or cost of proposed embodiments.

In an embodiment, the monitor unit may be adapted: to compare the first and second summary values to obtain a first comparison result; to generate a first weighted summary value based on the first measure of confidence; to generate a second weight summary value based on the second measure of confidence; to compare the first and second weighted summary values to obtain a second comparison result; and to determine a change in a capability or activity of the person based on the first and second comparison results. A relatively simple comparison process may therefore be implemented using conventional components. Similarly, the monitor unit may be adapted: to compare first and second characterising values to obtain a comparison result; and to determine a change in a capability or activity of the person further based on the comparison result.

Embodiments may further comprise a sensor adapted to detect, for each event occurrence, a value of a property of the person or the object controllable by the person and to generate a sensor output signal representative of the detected value. Preferably, the sensor may comprise at least one of: an accelerometer; a gyroscope; a movement sensor; a weight sensor; a pressure sensor; and a timing device. Further, the sensor may be adapted to be coupled to the person or the object. Preferably the object may comprise an item of furniture adapted to be used in the execution of an ADL.

For example, there exist many sensors that can be employed by an ADL monitoring system according to an embodiment. Typical sensors include PIR (Passive Infra-Red; measure movement and presence), OC (open-close; measure state of doors, in particular front doors, windows, and cupboards, including refrigerators), power sensors (measure current consumption of appliances, such as microwave, water cookers, TV, etc.); and pressure sensors or mats (measure occupancy of user sitting in chair, lying in bed, standing on door mat in front of front door, being at toilet, etc.). Many others exist and are conceivable, such as sensors to signal light switch state, or sensors that measure environmental conditions such as humidity, $CO_2$ level (or CO and smoke), Particulate Matter level, etc. A further range of sensors are those based on physical quantities, such as accelerometers, magnetometers, gyroscopes, and air pressure sensors. Accelerometers, for example, can also measure state of doors and their open-close movements or measure speed or velocity of movement of a person or an object moved by the person. Yet another range of sensors consists of microphones and cameras (including infra-red, or even UV and beyond, part of spectrum), to which also belong GPS and location-sensitive IR. Ultrasound or RF-based sensors, including RFID tagging, provide additional input. Appliances having an own IP-address, known as the internet-of-things, provide further sensor input signals that can be taken by the smart-home system.

Although the sensor(s) may be mounted in the monitoring environment (e.g. the person's home), they may also be attached to user utilities (such as a keyring) or put in clothes, in a pocket or bag, or as insole or undergarment, etc. They may also be fabricated to be worn explicitly like a wrist watch or pendant. Further, the sensors may communicate their output signals via a wired or wireless connection, or a combination thereof. Accordingly, in an embodiment, the sensor may be adapted to be coupled to the person or the object. The object may for example comprise an item of furniture (such as a fridge, cupboard, wardrobe, seat, door, white good, etc.) adapted to be used in the execution of an ADL.

Embodiments may therefore be implemented in conjunction with pre-existing, pre-installed or otherwise separately-provisioned presence sensors, and the output signals from such sensors may be received and processed in accordance with proposed concepts. Other embodiments may be provided with sensors (e.g. where appropriate sensors are not already available).

A sensor may also be adapted to undertake primary processing of the detected values, such a signal filtering, sampling, conditioning, etc., so as to reduce required transmission bandwidth and/or transmission duration for example. Alternatively, a sensor may send raw data.

The sensor arrangement/system may be positioned in a strategic position so that it detects the appropriate value without the person needing to intentionally or consciously activate/operate the sensor. In this way, a person may only need to undertake their normal activities. Such strategic positioning may ensure that a value of a property of the person or object can be automatically and accurately obtained, and this may not require the person to remember to undertake any special or additional activities in order for a value to be detected by the sensor. This may remove the risk of the person forgetting to activate a sensor (e.g. by pressing a button), for example.

Non-intrusive monitoring may therefore be realized with relatively simple sensors that provide data on specific properties/parameters of an object or properties of the person (such as movement, weight, speed, weight, and/or distance travelled for example). Such sensors for measuring ambient condition or properties/parameters of the object or environment may be simple, small and/or cheap. Also, the movement of the person may be detected with, for example, a Passive Infrared (PIR) sensor which is a cheap component. Movement sensors may be used to switch on lighting and people are therefore typically familiar with their usage.

Thus, monitoring systems of the invention may employ conventional sensors and/or existing sensor arrangements. Also, embodiments may employ sensors that are considered to be non-intrusive and more easily accepted by the monitored person. Yet, with the data provided by these sensors, changes in events may be determined and provide information on the person being monitored.

Such sensors may be employed by, or in conjunction with, embodiments so as to increase the number and/or accuracy of monitored events. They may also be used to confirm or qualify readings taken by a sensor, so that spurious or unintentional measurements are avoided. For example, signals from a location sensor worn by the monitored person may be used to confirm if weight readings taken by a weight sensing system are indeed attributable to the monitored person or some other person or animal (such as their pet), for example.

Embodiments may be further adapted to store the sensor output signal(s) or event data in a database adapted to store historical data relating to one or more previously detected changes and/or values of the property. Previously determined values of a property of the person or the object may therefore be stored, in a historical database for example, and then used in subsequent calculations. Furthermore, currently detected values and/or changes may be used to re-calculate or refine a previously determined trend.

It will be appreciated that all or part of the monitor unit may comprise one or more data processing units. For example, the monitor unit may be implemented using a single processor which is adapted to undertake data processing in order to determine a change in a physical or mental capability of the person (based on first and second summary values and first and second measures of confidence).

The monitor unit may be remotely located from the sensor(s), and a signal representative of detected values may be communicated to the monitor unit via a communication link.

The system may further comprise: a server device comprising the data processing unit, data confidence unit and the monitor unit; and a client device comprising the sensor. Dedicated data processing means may therefore be employed for the purpose of determining a change in the physical or mental capability of the person, thus reducing processing requirements or capabilities of other components or devices of the system.

The system may further comprise a client device, wherein the client device comprises the data processing unit, data confidence unit and monitor unit and a display system. In other words, a user (such as a care giver) may have an appropriately arranged client device (such as a laptop, tablet computer, mobile phone, PDA, etc.) which processes received event data in order to determine a change in the physical or mental capability of the person.

Thus, processing may be hosted at a different location from where the sensing happens. For example, for reasons of power efficiency (e.g. to improve battery lifetime) it might be advantageous to execute only part of the processing at the sensor location, thereby reducing associated costs, processing power, transmission requirements, etc.

Thus, it will be understood that processing capabilities may therefore be distributed throughout the system in different ways according to predetermined constraints and/or availability of processing resources.

Embodiments may also enable some of the processing load to be distributed throughout the system. For example, pre-processing may be undertaken at a sensor system. Alternatively, or additionally, processing could be undertaken at a communication gateway. In some embodiments, processing may be undertaken at a remote gateway or sever, thus relinquishing processing requirements from an end-user or output device. Such distribution of processing and/or hardware may allow for improved maintenance abilities (e.g. by centralising complex or expensive hardware in a preferred location). It may also enable computational load and/or traffic to be designed or located within a networked system according to the processing capabilities available. A preferable approach may be to process sensor signals locally and transmit extracted data for full processing at a remote server.

Activities of daily living concern basic activities that a person executes on a regular basis. Examples of activities of daily living are drinking/eating; cooking; medicating; sleeping; toileting; bathing; washing, any kind of exercising such as walking, leisure activities such as reading or TV watching and many more etc. Thus, the invention may provide a way to accurately monitor an ADL (such as cooking, eating, exercising, opening doors, etc.) in a simple and easy to implement manner.

The property of the person or the object may comprise at least one of: a velocity (e.g. translational and/or rotational) or speed of movement of the person or the object; a measure (such as magnitude, speed/rate of change, average, etc.) of force (or derivatives thereof) applied by the person to the object; a distance travelled by the object or a body part of the person; a rate of acceleration of the object or body part of the person; a reaction time of the person; a measure of posture of the person; a height of the person; and a bodyweight of the person. By detecting values of a property of a person and/or an object that the person interacts with, a change in the detected values over time may be identified and, from such changes, a physical or mental capability of a person may be monitored.

For example, a change (e.g. decrease) velocity or speed of movement of the person (as detected directly or inferred using an accelerometer affixed to the person, or as detected indirectly using a accelerometer integrated into an object that is moved by the person) may be used to identify and monitor the person's physical strength or speed, and further identify a future point in time where the person's physical strength or speed declines to a point where assistance or help will be required. By way of another example, a change (e.g. increase) in reaction time of the person (as detected using a timing device that detects an amount of elapsed time between a prompt or signal and a response from the person) may be used to identify and monitor the person's physical or mental capability in responding to a command, and further identify a future point in time where the person's capability will reduce to a level where additional assistance or help will be required. For example, predictive analytics may be employed to try and identify (and thus prevent or avoid) undesirable or worst case outcomes. This may help to provide cost savings (e.g. in a health care system) by timely enabling intervention. Embodiments may therefore be useful for identifying future requirements of a monitored person, and this may be achieved via extrapolation of changes determined by a proposed embodiment.

The monitor unit may be further adapted to generate an output signal based on the determined change.

In an embodiment, the monitor unit may be adapted to detect an irregularity based on a comparison of the determined change with a threshold value. For example, the monitor unit may employ a data processing unit that compares the determined change with a threshold value and then generates a warning signal if the determined change exceeds the threshold value. The threshold may be preprogramed and fixed, but it may be preferable to enable the threshold value to be set by a user preference. Also the threshold value may relate to a future value, and the trend may be extrapolated for comparison of an extrapolated value with the future threshold value. This may identify when a threshold value may be exceeded in the future, for example. By way of further example, the threshold value may be determined based on at least one of: one or more previously detected values of the property; and one or more previously determined changes in values of the property. In other words, the threshold may be defined by taking account of a history of detected values and/or a history of changes in values of the property so that it can be used to identify outlying values or anomalies.

Embodiments may therefore further comprise a user input interface adapted to receive a user input for defining or modifying one or more alert conditions, and the monitor unit may be adapted to generate an alert output signal based on the determined change and the one or more alert conditions.

Embodiments may be adapted to provide an output signal to at least one of: the person; a medical practitioner; and a caregiver.

Also, the threshold value may be enabled to be set to act on a person to be monitored or act on a group of persons to be monitored.

The monitor unit may be further adapted to generate a control signal for modifying a graphical element based on the determined change in the physical or mental capability of the person. Further, the system may further comprise a display system adapted to display the graphical element in accordance with the control signal generated by the monitor unit. In this way, a user (such as a care giver) may have an appropriately arranged display system that can receive and display information about the change in the physical or mental capability of the person, and that person may be remotely located from the user. Embodiments may therefore enable a user to remotely monitor a person using a portable display device, such as a laptop, tablet computer, mobile phone, PDA, etc.

According to another aspect of the invention, there is provided a method for monitoring activity of a person, wherein the method comprises: receiving event data relating to a plurality of occurrences of an event, the event data comprising, for each event occurrence, information representative of a detected value of a property of at least one of: the person; and an object controllable by the person; processing the received data to determine a first summary value for a first set of event occurrences that occurred during a first time period and to determine a second summary value for a second set of event occurrences that occurred during a second, different time period; determining first and second measures of confidence in the first and second summary values, respectively; and determining a change in a capability or activity of the person based on the first and second summary values and the first and second measures of confidence.

According to yet another aspect of the invention, there is provided computer program product for monitoring activity of a person, wherein the computer program product comprises a computer-readable storage medium having computer-readable program code embodied therewith, the computer-readable program code configured to perform all of the steps of an embodiment.

A computer system may be provided which comprises: a computer program product according to an embodiment; and one or more processors adapted to perform a method according to an embodiment by execution of the computer-readable program code of said computer program product.

In a further aspect the invention relates to a computer-readable non-transitory storage medium comprising instructions which, when executed by a processing device, execute the steps of the method of controlling a monitoring system display unit according to an embodiment.

These and other aspects of the invention will be apparent from and elucidated with reference to the embodiment(s) described hereinafter.

BRIEF DESCRIPTION OF THE DRAWINGS

Examples in accordance with aspects of the invention will now be described in detail with reference to the accompanying drawings, in which:

> Figure 1 is a simplified block diagram of a system for monitoring a person according to an embodiment;
> Figure 2A is a flow diagram of a method according to a proposed embodiment;
> Figure 2B is an exemplary flow diagram of the step 240 of the method of Figure 2A;
> Figures 3A-3D depict examples of an event property values for two different weeks, wherein, in Figures 3A-3D, the left plot shows detected property values for a subject during the first week after discharge, and the right plot data shows detected property values for the subject during the sixth week after discharge;
> Figure 4 is a simplified block diagram of a system for monitoring a person according to another embodiment; and
> Figure 5 is a simplified block diagram of a computer within which one or more parts of an embodiment may be employed.

DETAILED DESCRIPTION OF THE EMBODIMENTS

Proposed is a concept for monitoring a person, which may be useful for the purpose of unobtrusively monitoring the well-being or rehabilitation of the person for example. Such persons may, for instance, include a disabled person, an elderly person, an injured person, a medical patient, etc. Elderly persons can mean persons above 50 years, above 65 years, above 70, or above 80 years old, for example.

Illustrative embodiments may be utilized in many different types of monitoring environments, such as a hospital, ward, care home, person's home, etc. In order to provide a context for the description of elements and functionality of the illustrative embodiments, the Figures are provided hereafter as examples of how aspects of the illustrative embodiments may be implemented. It should therefore be appreciated the Figures are only examples and are not intended to assert or imply any limitation with regard to the environments, systems or methods in which aspects or embodiments of the present invention may be implemented.

In general, to be able to observe trends in "normal" daily behaviour of a person one may monitor ADLs of a person. From established trends, one may also spot unexpected activities, anomalies or deviations from expected values or patterns. The type of anomaly or irregularity can be different per case. A large class of physical or mental capabilities can relate to an ADL routine of the person. For example, a physical capability may be inferred from a parameter of an event occurrence, such as the speed by which a person opens/closes a door (or a cupboard, wardrobe, fridge, microwave, or other furnishings) or a drawer and/or the force applied to the door or drawer during the opening/closing process for example.

Embodiments of the present invention are directed toward enabling changes in a physical or mental capability of a person to be identified and potentially monitored. Such information may therefore be useful for monitoring the health or well-being of a person. Also, this may be used to generate an alert signal for an alerting or warning system that can indicate the person is in need of help, for example.

Embodiments employ the concept of using summary parameter values for sets of event occurrences from different time periods, along with measures of confidence in the summary parameter values, in order to distinguish meaningful changes in the parameter from noise or minor variations. By determining a measure of confidence (e.g. reliability measure) in detected values and/or summary values, embodiments may enable the identification of changes in a parameter of an event that are significant and representative of underlying changes in a person's physical or mental capability. This may help to reduce a number of false-positives (i.e. inaccurate or incorrect instances) and provide more accurate monitoring. Thus, embodiments may be useful for evaluation purposes, for example to assess if a subject shows a significant improvement in a physical or mental capability.

Physical or mental capabilities may be detected or inferred from sensor output signals and there already exist systems and methods for such detection or inference. Accordingly, the proposed concepts may be used in conjunction with existing ADL detection or monitoring systems/methods. For example, Dries Vermeiren et al describe a system based on 2 tri-axial accelerometers to detect the ADLs of a patient in a paper entitled "Detecting Human Motion: Introducing Step, Fall and ADL algorithms". Also, H Pirsiavas et al describe algorithms for detecting ADLs in first-person camera views in paper entitled "Detecting activities of daily living in first-person camera views" (CVPR, 2012). Because many such ADL detection or monitoring methods/systems are known and any one or more of these may be employed, detailed description of such methods/systems is omitted from this description.

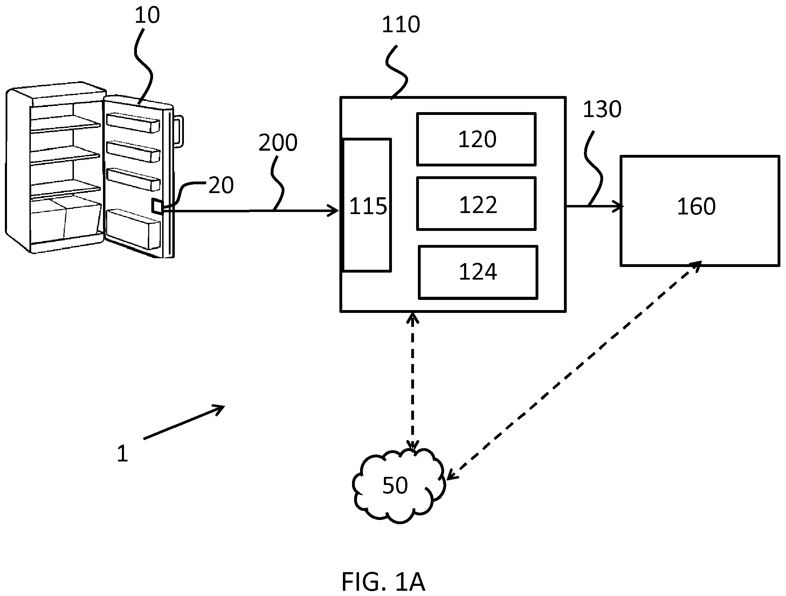

Fig. 1 shows an embodiment of a system 1 according to the invention comprising a fridge door 10 and a motion sensor 20 adapted to detect a velocity of the fridge door 10 which results from the person's usage (e.g. opening or closing) of the fridge door 10.

Here, the motion sensor 20 is situated integrated into the fridge door 10 so that it moves with the fridge door 10. For example, the motion sensor 20 may comprise an accelerometer, magnetometer, and gyroscope. A gyroscope is of particular interest when the fridge door 10 is a rotating one (i.e. hinged at one side to that it is rotated about a vertical axis when opened/closed) like that shown in Figure 1. Since a gyroscope measures rotation velocity, it can be mounted at any spot of the fridge door 10. For example, it can be mounted close to the hinge or rotation axis so that is hidden from sight, or it can be mounted far from the hinge (e.g. close to the handle of the fridge door shown in Figure 1) so that it can be accessed easily (for repair or replacement for example).

In this way, a person need only undertake their normal activities when using the fridge and may not even be aware that they are operating the motion sensor and being monitored. Such positioning (integrated into the fridge door 10, for example) may ensure that a property of the person or the fridge door 10 (such as: a velocity or speed of movement of the door 10; a magnitude of force applied by the person to the door 10; a distance travelled by the door 10; a rate of acceleration of the door 10; the duration of opening/closing of the door; the impulse (integrated force/acceleration); or the (peak) power (force/acceleration time velocity) exercised during an open and/or close event, for example) can be automatically and accurately obtained for each single usage event (e.g. opening or closing) of the fridge (e.g. every time the person accesses the contents of the fridge) without requiring the person to remember to undertake any special or additional activities in order for a property of the person or the fridge door 10 to be detected. For example, it can remove the need for a person to perform a specific additional action (e.g. pressing a button) in order to activate the motion sensor 20.

The motion sensor 20 comprises a motion sensing arrangement that is adapted to determine a velocity of movement of the fridge door 10, a distance travelled by the fridge door 10, and a rate of acceleration of the fridge door 10, when the fridge door 10 is opened. The motion sensor 20 may obtain numerous measurements before, during and after the person opens or closes the fridge door.

The motion sensor 20 is adapted to output sensor output signals 200 which are representative of the detected value(s) of the fridge door 10 during an opening or closing event. Of course, many more sensors may be employed so as to provide signals indicative of detected values of properties of the person and/or the fridge door. For example, the magnitude of a pulling or pushing force applied by the person to the fridge door may be detected using one or more pressure sensors. Such additional signals may be useful for identifying which of the sensor output signals 200 are indicative of a property of the person or the fridge door 10. They may also be used to confirm or qualify values detected by the motion sensor 20, so that spurious or unintentional measurements are avoided. For example, signals from a location sensor worn by the monitored person may be used to confirm if values detected by the motion sensor 20 are indeed attributable to the monitored person operating the fridge door 10, for example.

The motion sensor 20 communicates its output signals 200 via a wired or wireless connection. By way of example, the wireless connection may comprise a short-to-medium-range communication link. For the avoidance of doubt, short-to-medium-range communication link may be taken to mean a short-range or medium-range communication link having a range of up to around 100 meters. In short-range communication links designed for very short communication distances, signals typically travel from a few centimeters to several meters, whereas, in medium-range communication links designed for short to medium communication distances, signals typically travel up to 100 meters. Examples of short-range wireless communication links are ANT+, Bluetooth, Bluetooth low energy, IEEE 802.15.4, ISA100a, Infrared (IrDA), , Near Field Communication (NFC), RFID, 6LoWPAN, UWB, Wireless HART, Wireless HD, Wireless USB, ZigBee. Examples of medium-range communication links include Wi-Fi, ISM Band, Z-Wave. Here, the output signals are not encrypted for communication via the wired or wireless connection in a secured manner. However, it will be appreciated that, in other embodiment, one or more encryption techniques and/or one or more secure communication links may be employed for the communication of signals in the system.

The system further comprises a data processing system 110 having a signal interface 115 adapted to receive the sensor output signals 200. In this way, the signal interface of the data processing unit is adapted to receive event data relating to a plurality of occurrences of an event (e.g. usage event of opening or closing the fridge). The event data comprises, for each event occurrence, information representative of a detected value of a property of the person or the fridge door 10.

The data processing system 110 also comprises a data processing unit 120 that is adapted to process the received event data (e.g. from the received sensor output signals 200) to determine a first summary value for a first set of event occurrences that occurred during a first time period and to determine a second summary value for a second set of event occurrences that occurred during a second, different time period. In this example, the data processing unit 120 determines the first summary value based on an average (e.g. mean or median) value of the detected values of the property for the first set of event occurrences, and determines the second summary value based on an average value of the detected values of the property for the second set of event occurrences. For this purpose, the data processing unit 120 may communicate with one or more data processing resources available in the internet or "cloud" 50. Such data processing resources may undertake part or all of the processing required to determine a first summary value for a first set of event occurrences that occurred during a first time period and to determine a second summary value for a second set of event occurrences that occurred during a second, different time period.

The data processing system 110 also comprises a data confidence unit 122 adapted to determine first and second measures of confidence in the first and second summary values, respectively. In this example embodiment, the data confidence unit 122 is adapted to determine the first measure of confidence based on a standard deviation) of the detected values of the property for the first set of event occurrences (e.g. a standard error of the first summary value) and to determine the second measure of confidence based on a standard deviation of the detected values of the property for the second set of event occurrences (e.g. a standard error of the second summary value). Again, for this purpose, the data confidence unit 122 may communicate with one or more data processing resources available in the internet or "cloud" 50. Such data processing resources may undertake part or all of the processing required to determine first and second measures of confidence in the first and second summary values, respectively.

The data processing system 110 further comprises a monitor unit 124 adapted to determine a change in a capability or activity of the person based on the first and second summary values and the first and second measures of confidence. In particular, in the embodiment of Figure 1, the monitor unit 124 is adapted to compare the first and second summary values to obtain a first comparison result. The monitor unit 124 is also adapted to generate first and second weighted summary values based on the first and second measures of confidence, respectively, and to then compare the first and second weighted summary values to obtain a second comparison result. The monitor unit 124 then determines a change in a capability or activity of the person based on the first and second comparison results. Again, for this purpose, the data monitor unit 124 may communicate with one or more data processing resources available in the internet or "cloud" 50. Such data processing resources may undertake part or all of the processing required to determine a change in a capability or activity of the person based on the first and second summary values and the first and second measures of confidence.

Thus, it will be appreciated that the embodiment may employ distributed processing principles.

The data processing system 110 is further adapted to generate an output signal 130 representative of determined change in the capability or activity of the monitored person. In other words, after determine a change in a capability or activity of the person based on the first and second summary values and the first and second measures of confidence (either with or without communicating with data processing resources via the internet or "cloud"), an output signal 130 representative of or determined change is generated.

The system further comprises a graphical user interface (GUI) 160 for providing information to one or more users. The output signal 130 is provided to the GUI 160 via wired or wireless connection. By way of example, the wireless connection may comprise a short-to-medium-range communication link. As indicated in Figure 1, the output signal 130 is provided to the GUI 160 from the data processing unit 110. However, where the system, has made use of data processing resources via the internet or cloud 50), an output signal may be made available to the GUI 160 via the internet or cloud 50.

Based on the output signal 130, the GUI 160 is adapted to communicate information by displaying one or more graphical elements in a display area of the GUI 160. In this way, the system may communicate information about a change in the capability or activity of the monitored person that may be useful for indicating that the person is in need of attention or for estimating when the person may be expected to require assistance or attention. For example, the GUI 160 may be used to display graphical elements to a medical practitioner, a caregiver, a family member or close relative. Alternatively, or in addition, the GUI 160 may be adapted to display graphical elements to the monitored person.

From the above description of the embodiments of Figure 1, it will be understood that there is proposed a system for identifying a change in a capability or activity of the person that can be considered to comprise four main sub-systems/functions: (i) The first is a source of event data - this may, for example, comprise a wearable sensor that can detect one or more properties of events (such as door usage, walking bouts or chair rises for example); (ii) The second implements a function for summarizing the properties of an event over all event occurrences during a certain period (for example 1 day) - this function may be, for example, determine a mean value, a median value, a certain percentile or the inter-quartile range; (iii) The third implements a function matching with the summarizing function, to determine a confidence measure of the summary value(s); and (iv) The fourth implements an algorithm that filters out non-significant changes, and flags only significant changes by analysing the summary value(s) and the confidence measure(s).

The first and second sub-systems/functions can provide the periodic (e.g. daily) summary features, and can be chosen as suitable for intended application.

The third sub-system/function, which determines the confidence level of the summary values, preferably depends on the summary function used. For example, to determine the confidence level of the mean, the standard error of the mean shall be used. For the median, an analogous function "standard error of the median" shall be used. For alternative summary functions, matching standard-error functions may be selected or developed. Also, the confidence level can be adapted depending on the required sensitivity / specificity.

The fourth sub-system/function is implemented to distinguish significant changes from non-significant changes (e.g. to filter out noise or small variations). To filter out non-significant changes, a combination of variance of the summary values and measures of confidence summary values are used. For instance, the following two tests are performed, and if both tests indicate a change, the change can be flagged as detected change.

Test 1

The first test is on the daily summary values. To test if the daily summary values indicate a change, a two sample test can be used, comparing the summary values of the first period with those of the second period.

Test 2

The second test uses the daily summary values, as well as the measures of confidence (e.g. confidence intervals such a standard error). First, a combined estimate with measures of confidence is estimated from a set of daily summary values with measures of confidence. The summary value and the distances to the lower and higher bound are determined as weighted mean and root-sum-squares, respectively, of the daily summary values and the daily distances to the lower and higher bounds. The reciprocals of the widths of the confidence intervals, and of the distances to the lower and higher bounds are used as the weights. Given a set of N days with lower bound, estimate and higher bound ($1_i$, $e_i$, $h_i$), a combined set (L,E,H) is estimated as indicated by the following three equations 1:

$$E = \sum_{i=1}^{N} w_{e,i} \cdot e_i$$

$$L = E - \sqrt{\sum_{i=1}^{N} w_{l,i} \cdot (e_i - l_i)^2}$$

$$H = E + \sqrt{\sum_{i=1}^{N} w_{h,i} \cdot (h_i - e_i)^2}$$

Further, weights $w_i$ are estimated from a set of distances $d_i$ using the following equation:

$$w_i = \frac{1}{W} \frac{1}{d_i + \frac{1}{2} d_{med}}$$

wherein

$$W = \sum_{i=1}^{N} \frac{1}{d_i + \frac{1}{2} d_{med}}_i$$

and $d_{med}$ is the median of the values $d_i$. For calculation of the weights $w_{e,i}$, $w_{l,i}$ and $w_{h,i}$, the distances $h_i - l_i$, $e_i - l_i$, and $h_i - e_i$, respectively, are used.

For the two periods to be compared, combined sets $(L_1, E_1, H_1)$ and $(L_2, E_2, H_2)$ are determined. Depending on which of the two estimates $E$ is higher, a difference-score $z$ is calculated as:

$$z = \frac{E_2 - E_1}{\sqrt{(H_1 - E_1)^2 + (E_2 - L_2)^2}}, E_2 > E_1$$

or

$$z = \frac{E_1 - E_2}{\sqrt{(E_1 - L_1)^2 + (H_2 - E_2)^2}}, E_1 > E_2$$

If the difference score is larger than 1, the second test indicates a change.

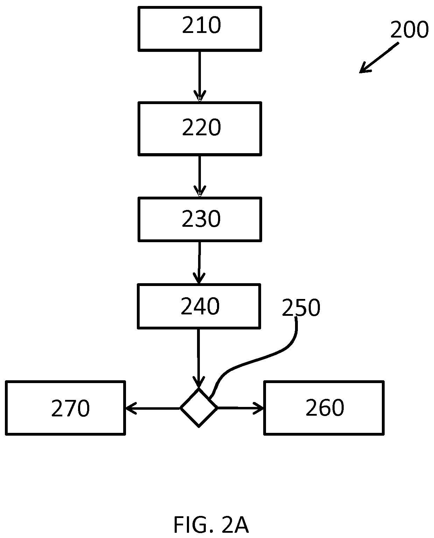

Referring now to Figure 2A, there is shown a flow diagram of an exemplary method 200 for monitoring a physical or mental capability of a person.

The method begins with step 210 in which event data relating to a plurality of occurrences of an event is received. The event data comprises, for each event occurrence, information representative of a detected value of a property of the person or an object manipulated by the person. In this example, the event occurrence walking between two rooms and the property is the time taken to complete the event of walking between two rooms. Thus, detected values of the time taken may be provided by presence sensors situated in the two rooms for example.

Next, in step 220, the received event data is processed to determine a first summary value for a first set of event occurrences that occurred during a first time period (e.g. a first week) and to determine a second summary value for a second set of event occurrences that occurred during a second, different time period (e.g. a second, subsequent week). Here, the step 220 comprises determining the first summary value based on a percentile range of the detected values of the time taken for the first set of event occurrences, and determining the second summary value based on a percentile range of the detected values of the time taken for the second set of event occurrences.

Then, in step 230, first and second measures of confidence in the first and second summary values are determined, respectively. This may be done, for example, by analysing all of the detected values for first set of event occurrences (i.e. all detected values of time taken to walk between the two rooms during the first week) and determining a measure of confidence based on the standard deviation of the detected values for first set of event occurrences. Likewise, all of the detected values for second set of event occurrences (i.e. all detected values of time taken to walk between the two rooms during the second, subsequent week) may be analysed and then a measure of confidence determined based on the standard deviation of the detected values for second set of event occurrences.

Next, in step 240, a change in a capability or activity (e.g. walking capability) of the person is determined based on the first and second summary values and the first and second measures of confidence.

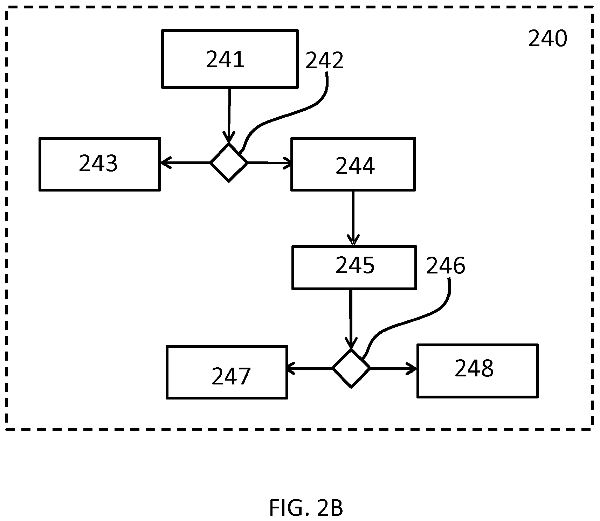

Referring now to Figure 2B, the determination process of step 240 is shown in more detail. The process begins with step 241 comparing the first and second summary values (from step 220) to obtain a first comparison result (e.g. difference value) representative of a level of agreement between the first and second summary vales. This, for example, may comprise implementation of Test 1 detailed above.

The process then proceeds to step 242 wherein the first comparison result is compared to a threshold to see if the level of agreement between the first and second summary values is above a predetermined threshold level. If it is (i.e. if there is agreement between the first and second summary values), the method proceeds to step 243 wherein it is determined that there is no significant change in the capability or activity (e.g. walking capability) of the person. If, however, it is determined that the level of agreement between the first and second summary values is not above a predetermined threshold level (i.e. if there is not agreement between the first and second summary values), the method proceeds to step 244.

In step 244, first and second weighted summary values are generated based on the first and second measures of confidence (from step 230), respectively. Next, in step 245, the first and second weighed summary values are compared to obtain a second comparison result representative of a level of agreement between the first and second weighted summary vales. This, for example, may comprise implementation of Test 2 detailed above. The process then proceeds to step 246 wherein the second comparison result is compared to a threshold to see if the level of agreement between the first and second weighted summary values is above a predetermined threshold level. If it is (i.e. if there is agreement between the first and second weighted summary values), the method proceeds to step 247 wherein it is determined that there is no significant change in the capability or activity (e.g. walking capability) of the person. If, however, it is determined that the level of agreement between the first and second weighted summary values is not above a predetermined threshold level (i.e. if there is not agreement between the first and second weighted summary values), the method proceeds to step 248 wherein it is determined that there is a significant change in the capability or activity (e.g. walking capability) of the person.

Referring now back to Figure 2A, after completion of step 220, the method continues to step 260 wherein any detected change is compared with a predetermined threshold value. The threshold value can be preprogramed, fixed or dynamically set in response to calculations based on one or more previously obtained values (e.g. using trend analysis), but is preferably also enabled to be set by a user preference. Thus, the threshold may be based on previously determined changes values. In other words, the threshold may be defined by taking account of a history of the person and/or taking account of previous calculations so that it can be used to identify changes that may be of particular importance or interest.

If, in step 250, a change is determined to exceed the first threshold value, the method proceeds to step 260 wherein a warning signal is generated and output along with information describing the detected change (e.g. in walking speed of the person). If, in step 250, the change is determined to not exceed the first threshold value, the method proceeds to step 270 wherein the information describing the detected change is output without any warning signal.

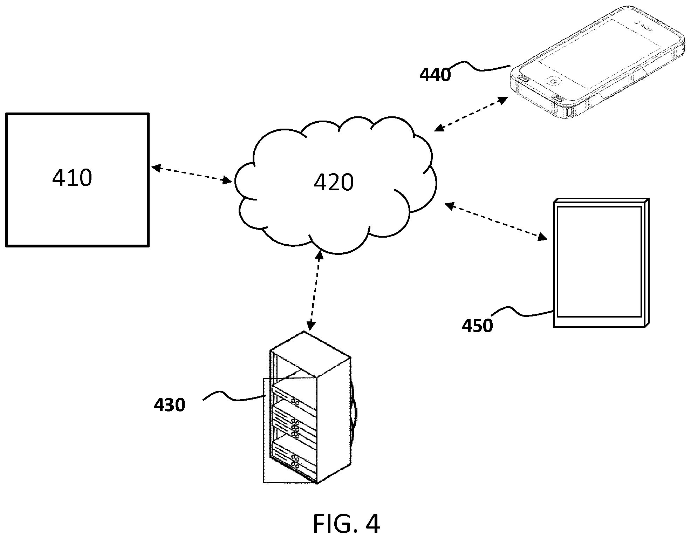

Thus, by way of example, by way of example, one or more steps of the method 200 for monitoring a person may be implemented in a portable computing device (such as the smartphone or portable computer shown in Figure 4) in order to control the display of graphical elements on a display.

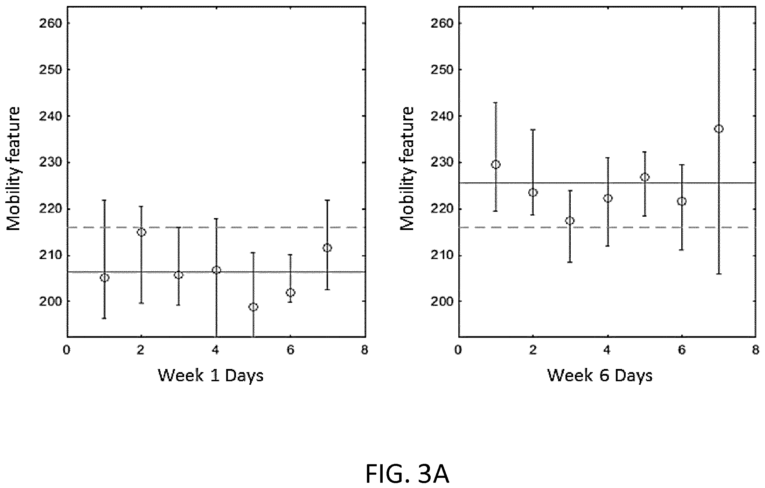

Referring now to Figure 3A-3D there are shown examples of an event property (namely a mobility feature) being compared between two different weeks. In Figures 3A-3D, the left plot shows detected property values for a subject (e.g. patient) during the first week after discharge, and the right plot data shows detected property values for the subject during the sixth week after discharge. The solid line indicates the mean (i.e. summary value) of the week, and the dashed line indicated the mean over the two weeks being compared. Error bars indicate the 95% confidence interval, and thus represent a measure of confident in each detected property value.

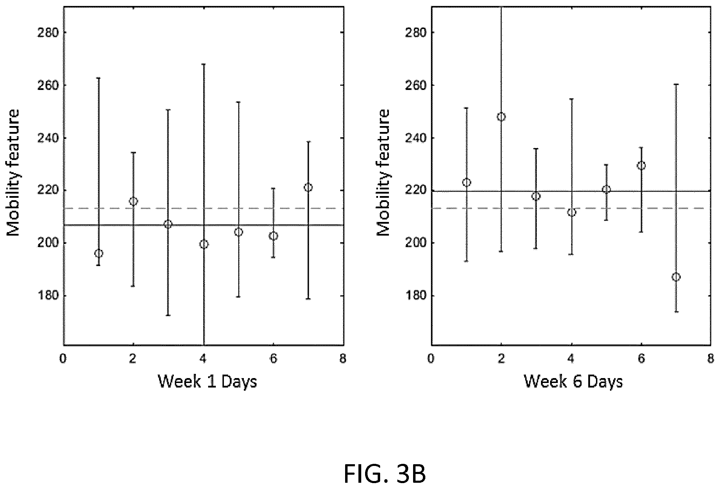

A first example is depicted in Figure 3A and employs all data for an event property for the analysis. Conversely, a second example is depicted in Figure 3B and employs only 15% of the data for an event property (i.e. data for 85% of event occurrence is discarded). From a comparison of the plots in Figures 3A and 3B, it can be seen that there is increased uncertainty of the property values (represented by the longer vertical lines which depict the larger/wider 95% confidence interval). Due to the increased uncertainty of the property values in the second example, analysis of the second would result in the example of Figure 3B not being considered a change. This demonstrates the effect/impact of discarding data relating to a large portion of detected event occurrences.

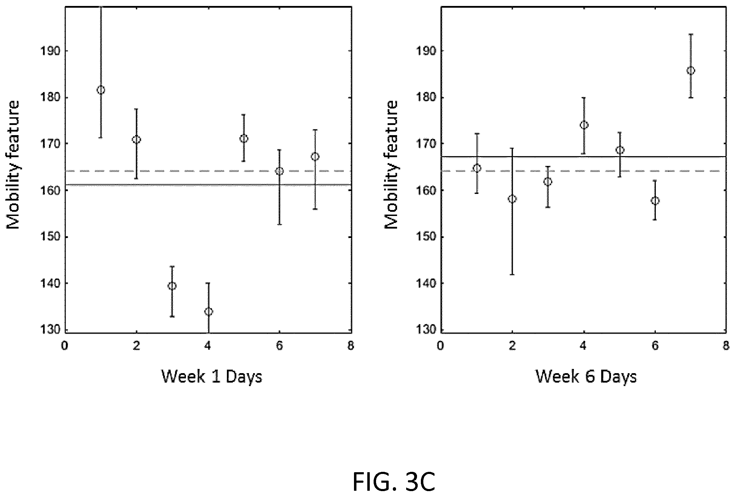

A third example is depicted in Figure 3C shows data having a high precision (i.e. low uncertainty represented by the shorter vertical lines which depict the smaller/narrower 95% confidence interval) on a daily basis, but still fluctuating between days. In this case, the second test which employs measures of confidence information, would indicate a change. Conversely, because of the variation between days, the first test which simply compares the mean values (i.e. summary values) for the two weeks will not indicate a change.

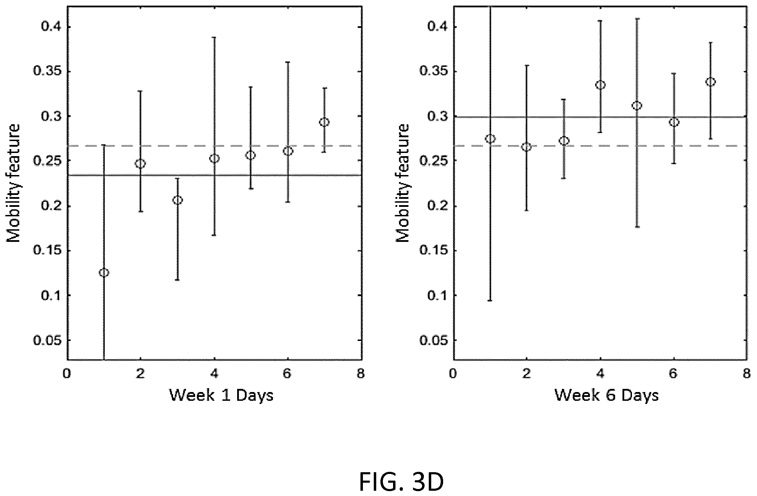

Finally, a fourth example is depicted in Figure 3D which is just the opposite situation than in the Figure 3C. Thus, Figure 3D shows data having a low precision (i.e. high uncertainty represented by the longer vertical lines which depict the larger/wider 95% confidence interval) on a daily basis, but with only minor fluctuation between days. In this case, the first test which simply compares the mean values (i.e. the summary values) for the two weeks does indicate a change. However, due to the large uncertainty of the daily values, the second test which incorporates the measured of confidence (e.g. the confidence intervals) will not indicate a change.

Accordingly, it will be appreciated that, by determining a change based on the summary values and the measures of confidence, proposed embodiments can provide more accurate identification of significant changes, and thus filter out or reduce non-significant changes.

Referring now to Fig. 4, there is depicted another embodiment of a system according to the invention comprising an accelerometer arrangement 410 adapted to detect movement of the person. Here, the accelerometer arrangement 410 comprises a high-resolution tri-axis accelerometer arrangement 410 adapted to be integrated into a necklace or pendant that is worn by the person. The accelerometer arrangement 410 is adapted to output one or more signals which are representative of the detected value(s) of a person's movement.

Although this embodiment has been described as employing a portable and wearable sensor arrangement, it will be understood that, in alternative embodiments, the movement of the person may be detected using one or more sensors strategically positioned within a monitoring environment. For example, a movement detection system may be positioned at the entrance or doorway to a room so as to measure a person's speed upon entering and leaving the room. Further, where multiple locations may be provided (e.g. on first and second floors, etc.), multiple movement sensing systems may be employed and the measurements combined.

The accelerometer arrangement 410 communicates the output signals via the internet 420 (using a wired or wireless connection for example) to a remotely located data processing system 430 (such as server).

The data processing system 430 is adapted to receive the one or more output signals from the accelerometer arrangement 410 and process the received signal(s) in accordance with a method according to a proposed embodiment to determine a change in a movement capability of the person. More specifically, the method processes the received sensor output signal(s) determine a first summary value for a first set of event occurrences that occurred during a first time period and to determine a second summary value for a second set of event occurrences that occurred during a second, different time period. The method also determines first and second measures of confidence in the first and second summary values, respectively. Based on the first and second summary values and the first and second measures of confidence, the method determines a change in a movement capability of the person.

The data processing system 430 is further adapted to generate output signals representative of a determined change in movement capability of the person. Thus, the data processing 430 provides a centrally accessible processing resource that can receive information from the accelerometer arrangement 410 and run one or more algorithms to transform the received information into a description of a movement capability of the person. Information relating to the movement capability can be stored by the data processing system (for example, in a database) and provided to other components of the system. Such provision of information about a detected or inferred change in a movement capability of the person may be undertaken in response to a receiving a request (via the internet 420 for example) and/or may be undertaken without request (i.e. 'pushed').

For the purpose of receiving information about a detected or inferred change in a movement capability of the person from the data processing system, and thus to enable the person's mobility to be monitored, the system further comprises first 440 and second 450 mobile computing devices.

Here, the first mobile computing device 440 is a mobile telephone device (such as a smartphone) with a display for displaying graphical elements representative of a person's physical or mental well-being. The second mobile computing device 450 is a mobile computer such as a Laptop or Tablet computer with a display for displaying graphical elements representative of a person's mobility.

The data processing system 430 is adapted to communicate output signals to the first 440 and second 450 mobile computing devices via the internet 420 (using a wired or wireless connection for example). As mentioned above, this may be undertaken in response to receiving a request from the first 440 or second 450 mobile computing devices.

Based on the received output signals, the first 440 and second 450 mobile computing devices are adapted to display one or more graphical elements in a display area provided by their respective display. For this purpose, the first 440 and second 450 mobile computing devices each comprise a software application for processing, decrypting and/or interpreting received output signals in order to determine how to display graphical elements. Thus, the first 440 and second 450 mobile computing devices each comprise a processing arrangement adapted to one or more values representative of the person's mobility, and to generate a display control signal for modifying at least one of the size, shape, position, orientation, pulsation or colour of the graphical element based on the one or more values representative of mobility.

The system can therefore communicate information about an inferred or detected change in a movement capability of the person to users of the first 440 and second 450 mobile computing devices. For example, each of the first 440 and second 450 mobile computing devices may be used to display graphical elements to a medical practitioner, a caregiver, a family member or close relative.

Implementations of the system of Figure 4 may vary between: (i) a situation where the data processing system 430 communicates display-ready bodyweight trend data, which may for example comprise display data including graphical elements (e.g. in JPEG or other image formats) that are simply displayed to a user of a mobile computing device using conventional image or webpage display (which can be web based browser etc.); to (ii) a situation where the data processing system 430 communicates raw data set information that the receiving mobile computing device then processes to determine a change in a movement capability of the person, and then displays graphical elements based on the determined change (for example, using local software running on the mobile computing device). Of course, in other implementations, the processing may be shared between the data processing system 430 and a receiving mobile computing device such that part of the data generated at data processing system 430 is sent to the mobile computing device for further processing by local dedicated software of the mobile computing device. Embodiments may therefore employ server-side processing, client-side processing, or any combination thereof.

Further, where the data processing system 430 does not 'push' information (e.g. output signals), but rather communicates information in response to receiving a request, the user of a device making such a request may be required to confirm or authenticate their identity and/or security credentials in order for the information to be communicated.

It is also noted that, although it has been described above that embodiments need not employ additional/supplementary sensors, some embodiments may further comprise a sensor adapted to detect a value of a property of at least one of: the environment, control/operation of an object, and the monitored person. Such a supplementary sensor arrangement may help to improve the accuracy of speed determination for example. Supplementary sensor readings may, for instance, qualify or refine data analysis undertaken by the signal processing unit and/or the monitor unit. For instance, the person may wear or carry an identification tag or location tracker which can enable embodiments to distinguish and disregard the presence of other people or animals detected by the presence sensors. By way of further example, signals from a location sensor worn by the monitored person may be used to confirm if presence detections are indeed attributable to the monitored person or some other person.

The presence sensors may be arranged in strategic locations/positions so as to detect a person's presence without the person needing to intentionally or consciously activate/operate the sensors. In this way, a person may only need to undertake their normal activities. Such strategic positioning may ensure that presence of the person can be automatically and accurately detected, and this may not require the person to remember to undertake any special or additional activities in order for a value to be detected by the sensor. This may remove the risk of the person forgetting to activate a sensor (e.g. by pressing a button), for example.

There exist many sensors that can be employed by embodiments. Typical sensors include PIR (pyroelectric infrared sensor; detect movement and presence) or pressure sensors. Many others exist and are conceivable, such as sensors comprising microphones and cameras (including infra-red (IR), or even UV and beyond, part of spectrum).

The sensors may also be adapted to undertake primary processing of the detected values, such a signal filtering, sampling, conditioning, etc., so as to reduce a required transmission bandwidth and/or transmission duration for example.

Non-intrusive monitoring may therefore be realized with relatively simple sensors that provide data on specific properties of the person (such as movement, for example). Also, the movement of the person may be detected with sensors that are cheap and widely employed. For instance, movement sensors may be used to switch on lighting and people are therefore typically familiar with their usage. Thus, embodiments may employ sensors that are considered to be non-intrusive and more easily accepted by the monitored person. Yet, with the data provided by these sensors, a person's speed may be accurately determined and provide more information on the person being monitored. Thus, some embodiments of the invention may employ conventional sensors and/or existing sensor arrangements. Also, embodiments may employ sensors that are considered to be non-intrusive and more easily accepted by the monitored person.

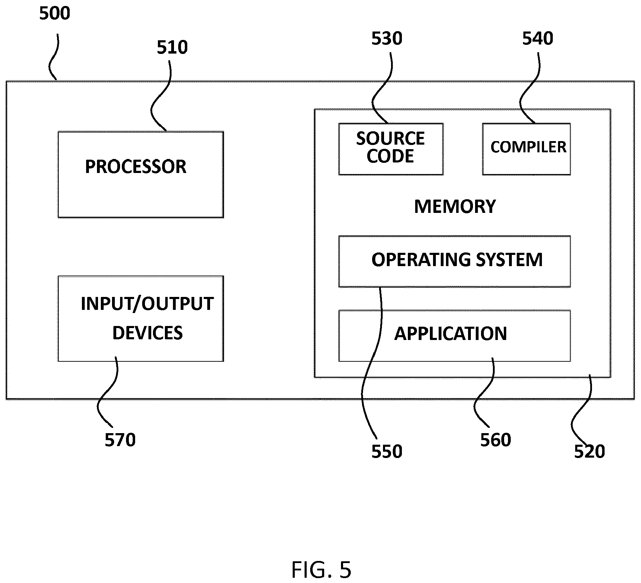

Figure 5 illustrates an example of a computer 500 within which one or more parts of an embodiment may be employed. Various operations discussed above may utilize the capabilities of the computer 500. For example, one or more parts of a monitoring system adapted to monitor a person may be incorporated in any element, module, application, and/or component discussed herein.

The computer 500 includes, but is not limited to, PCs, workstations, laptops, PDAs, palm devices, servers, storages, and the like. Generally, in terms of hardware architecture, the computer 500 may include one or more processors 510, memory 520, and one or more I/O devices 570 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and nonvolatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 540, source code 530, and one or more applications 560 in accordance with exemplary embodiments. As illustrated, the application 560 comprises numerous functional components for implementing the features and operations of the exemplary embodiments. The application 560 of the computer 500 may represent various applications, computational units, logic, functional units, processes, operations, virtual entities, and/or modules in accordance with exemplary embodiments, but the application 560 is not meant to be a limitation.

The operating system 550 controls the execution of other computer programs, and provides scheduling, input-output control, file and data management, memory management, and communication control and related services. It is contemplated by the inventors that the application 560 for implementing exemplary embodiments may be applicable on all commercially available operating systems.

Application 560 may be a source program, executable program (object code), script, or any other entity comprising a set of instructions to be performed. When a source program, then the program is usually translated via a compiler (such as the compiler 540), assembler, interpreter, or the like, which may or may not be included within the memory 520, so as to operate properly in connection with the O/S 550. Furthermore, the application 560 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, php. Python, ASP scripts, FORTRAN, COBOL, Perl, Java, ADA, .NET, and the like.

The I/O devices 570 may include input devices such as, for example but not limited to, a mouse, keyboard, scanner, microphone, camera, etc. Furthermore, the I/O devices 570 may also include output devices, for example but not limited to a printer, display, etc. Finally, the I/O devices 570 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 570 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at startup, start the O/S 550, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 510 is configured to execute software stored within the memory 520, to communicate data to and from the memory 520, and to generally control operations of the computer 500 pursuant to the software. The application 560 and the O/S 550 are read, in whole or in part, by the processor 510, perhaps buffered within the processor 510, and then executed.

When the application 560 is implemented in software it should be noted that the application 560 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 560 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Java, Smalltalk, C++ or the like, and conventional procedural programming languages, optimized for embedded implementation, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

From the above description, it will be appreciated that embodiments may therefore be useful for monitoring of elderly, disabled or unwell individuals so to support independent living. Detected changes can be used both for real-time monitoring and alerts, as well as to detect deviations from usual or expected patterns or trends.

Embodiments may be supported by the current and future trends towards smart-homes or connected lighting.

The description has been presented for purposes of illustration and description, and is not intended to be exhaustive or limited to the invention in the form disclosed. Many modifications and variations will be apparent to those of ordinary skill in the art. Embodiments have been chosen and described in order to best explain principles of proposed embodiments, practical application(s), and to enable others of ordinary skill in the art to understand that various embodiments with various modifications are contemplated.

## Claims

1. A system (110) for monitoring activity of a person, wherein the system comprises:

   a signal interface (115) adapted to receive event data relating to a plurality of occurrences of an event, the event data comprising, for each event occurrence, information representative of a detected value of a property of at least one of: the person; and an object controllable by the person; a data processing unit (120) adapted to process the received event data to determine a first summary value for a first set of event occurrences that occurred during a first time period and to determine a second summary value for a second set of event occurrences that occurred during a second, different time period; a data confidence unit (122) adapted to determine first and second measures of confidence in the first and second summary values, respectively; and a monitor unit (124) adapted to determine a change in a capability or activity of the person based on the first and second summary values and the first and second measures of confidence.

2. The system of claim 1, wherein the data processing unit (120) is further adapted to determine a first characterising value for the first and second sets of event occurrences based on the first and second summary values, to determine a third summary value for a third set of event occurrences that occurred during a third time period, to determine a fourth summary value for a fourth set of event occurrences that occurred during a fourth time period, and to determine a second characterising value for the third and fourth sets of event occurrences based on the third and fourth summary values, wherein the data confidence unit (122) is adapted to determine third and fourth measures of confidence in the third and fourth summary values, respectively, and wherein the monitor unit (124) is adapted to determine a change in a capability or activity of the person based on the first and second characterising values and the first to fourth measures of confidence.

3. The system of claim 2, wherein the monitor unit (124) is adapted:

   to compare the first and second characterising values to obtain a primary comparison result; to generate a first weighted characterising value based on the first and second summary values and the first and second measures of confidence; to generate a second weight characterising value based on the third and fourth summary values and the third and fourth measures of confidence; to compare the first and second weighted characterising values to obtain a secondary comparison result; and to determine a change in a capability or activity of the person based on the primary and secondary comparison results.

4. The system of claim 1 or 2, wherein the data processing unit (120) is adapted to determine the first summary value based on an average value of the detected values of the property for the first set of event occurrences, and to determine the second summary value based on an average value of the detected values of the property for the second set of event occurrences.

5. The system of any preceding claim, wherein the data processing unit (120) is adapted to determine the first summary value based on a percentile range of the detected values of the property for the first set of event occurrences, and to determine the second summary value based on a percentile range of the detected values of the property for the second set of event occurrences.

6. The system of any preceding claim, wherein the data confidence unit (122) is adapted to determine the first measure of confidence based on a standard deviation of the detected values of the property for the first set of event occurrences and to determine the second measure of confidence based on a standard deviation of the detected values of the property for the second set of event occurrences.

7. The system of claim 6, wherein the data confidence unit (122) is adapted to determine the first measure of confidence based on a standard error of the first summary value and to determine the second measure of confidence based on a standard error of the second summary value.

8. The system of any preceding claim, wherein the monitor unit (124) is adapted:

to compare the first and second summary values to obtain a first comparison result;
to generate a first weighted summary value based on the first measure of confidence;
to generate a second weight summary value based on the second measure of confidence;
to compare the first and second weighted summary values to obtain a second comparison result; and
to determine a change in a capability or activity of the person based on the first and second comparison results.

9. The system of any preceding claim, further comprising:

a sensor (20) adapted to detect, for each event occurrence, a value of a property of the person or the object (10) controllable by the person and to generate a sensor output signal (200) representative of the detected value,
preferably wherein the sensor (20) comprises at least one of: an accelerometer; a gyroscope; a movement sensor; a weight sensor; a pressure sensor; and a timing device.

10. The system of any preceding claim, wherein the property comprises at least one of: a velocity of movement of the person or the object; a measure of force applied by the person to the object; a distance travelled by the object or a body part of the person; a rate of acceleration of the object or body part of the person; a height of the person; a measure of posture of the person; a bodyweight of the person; and a reaction time of the person.

11. The system of any preceding claim, wherein the sensor (20) is adapted to be coupled to the person or the object (10), and preferably wherein the object comprises an item of furniture adapted to be used in the execution of an ADL.

12. The system of any preceding claim, wherein the monitor unit (124) is adapted to detect an irregularity based on a comparison of the determined change with a threshold value,
and preferably wherein the threshold value is determined based on at least one of: one or more previously detected values of the property; and one or more previously determined changes in values of the property.

13. The system of any preceding claim, wherein the monitor unit (124) is further adapted to generate a control signal (130) for modifying a graphical element based on the determined change in the physical or mental capability of the person,
and wherein the system further comprises a display system (160) adapted to display the graphical element in accordance with the control signal generated by the monitor unit.

14. A method (200) for monitoring activity of a person, wherein the method comprises:

receiving (210) event data relating to a plurality of occurrences of an event, the event data comprising, for each event occurrence, information representative of a detected value of a property of at least one of: the person; and an object controllable by the person;
processing (220) the received event data to determine a first summary value for a first set of event occurrences that occurred during a first time period and to determine a second summary value for a second set of event occurrences that occurred during a second, different time period;
determining (230) first and second measures of confidence in the first and second summary values, respectively; and
determining (240) a change in a capability or activity of the person based on the first and second summary values and the first and second measures of confidence.

15. A computer program product comprising computer readable code storable on, or stored on, or downloadable from a communications network, which code when run on a computer implements the methods of claim 14.

FIG. 1A

EP 3 477 653 A1

FIG. 2A

FIG. 2B

EP 3 477 653 A1

FIG. 3A

FIG. 3B

FIG. 3C

FIG. 3D

FIG. 4

FIG. 5

Europäisches
Patentamt

European
Patent Office

Office européen
des brevets

# EUROPEAN SEARCH REPORT

Application Number

EP 17 19 8691

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2017/045025 A1 (COMMW SCIENT IND RES ORG [AU]) 23 March 2017 (2017-03-23) <br> * paragraphs [0071] - [0075] * <br> * abstract * <br> * paragraphs [0096] - [0102], [0118] * <br> ----- | 1-15 | INV. <br> G16H40/63 <br> G16H20/30 |
| Y | WO 2016/135382 A1 (JOUZEN OY [FI]) 1 September 2016 (2016-09-01) <br> * abstract * <br> * paragraphs [0007], [0008], [0094] - [0099] * <br> ----- | 1-15 | |
| Y | US 2014/128784 A1 (WILEY RONALD L [US]) 8 May 2014 (2014-05-08) <br> * paragraphs [0019], [0057] - [0059], [0088] - [0090] * <br> ----- | 1-15 | |
| A | US 2011/165998 A1 (LAU JACK [HK] ET AL) 7 July 2011 (2011-07-07) <br> * paragraphs [0025] - [0045] * <br> ----- | 1-15 | TECHNICAL FIELDS SEARCHED (IPC) |
| A | WO 2016/103197 A1 (PERFORMANCE LAB TECHNOLOGIES LTD [NZ]) 30 June 2016 (2016-06-30) <br> * pages 13-26 * <br> * pages 31,49 * <br> ----- | 1-15 | G16H <br> A63B <br> A61B |

The present search report has been drawn up for all claims

| Place of search | Date of completion of the search | Examiner |
|---|---|---|
| Munich | 12 April 2018 | Rivera Pons, Carlos |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 19 8691

This annex lists the patent family members relating to the patent documents cited in the above-mentioned European search report.
The members are as contained in the European Patent Office EDP file on
The European Patent Office is in no way liable for these particulars which are merely given for the purpose of information.

12-04-2018

| Patent document cited in search report | | | Publication date | Patent family member(s) | | | Publication date |
|---|---|---|---|---|---|---|---|
| WO 2017045025 | A1 | | 23-03-2017 | NONE | | | |
| WO 2016135382 | A1 | | 01-09-2016 | EP | 3262548 | A1 | 03-01-2018 |
| | | | | US | 2018042540 | A1 | 15-02-2018 |
| | | | | WO | 2016135382 | A1 | 01-09-2016 |
| US 2014128784 | A1 | | 08-05-2014 | NONE | | | |
| US 2011165998 | A1 | | 07-07-2011 | NONE | | | |
| WO 2016103197 | A1 | | 30-06-2016 | NONE | | | |

EPO FORM P0459

For more details about this annex : see Official Journal of the European Patent Office, No. 12/82